# EUROPÄISCHE PATENTSCHRIFT

(11) **EP 1 872 823 B1**
(45) Veröffentlichungstag und Bekanntmachung des Hinweises auf die Patenterteilung: **06.10.2010**
(21) Anmeldenummer: 07012576.0
(22) Anmeldetag: 27.06.2007
(51) Int. Cl.: A61M 37/00, A01K 11/00

(54) **Anordnung zum wiederholten Aufstechen einer Haut mit einem Handgerät und Nadelmodul**
Assembly for repeated punctuation of skin with a hand device and needle module
Agencement destiné à la piqûre répétée de la peau avec un appareil manuel et module d'aiguille

(30) Priorität: 28.06.2006 EP 06013344
(43) Veröffentlichungstag der Anmeldung: 02.01.2008
(73) Patentinhaber: MT Derm GmbH, 14167 Berlin (DE)
(72) Erfinder: Kluge, Jörn, 14513 Teltow (DE); Podolski, Denis, 10961 Berlin (DE); Plückhahn, Kristian, 12437 Berlin (DE)
(74) Vertreter: Bittner, Thomas L.

(56) Entgegenhaltungen:
- EP-A1- 1 118 919
- EP-A1- 1 163 974
- EP-A2- 0 269 562
- WO-A-2005/110303
- DE-A1- 10 118 034
- DE-U1- 29 923 931
- FR-A- 2 023 246
- US-A1- 2004 220 602
- US-A1- 2006 129 140
- US-B1- 6 345 553

## Beschreibung

Die Erfindung bezieht sich auf eine Anordnung zum wiederholten Aufstechen einer Haut mit einem Handgerät, welches ein Antriebsmodul mit einer Antriebsvorrichtung und ein Nadelmodul aufweist, sowie ein Nadelmodul für eine solche Anordnung.

### Hintergrund der Erfindung

Anordnungen zum wiederholten Aufstechen einer Haut mit einem Handgerät, welches ein Antriebsmodul mit einer Antriebsvorrichtung, die konfiguriert ist, um eine Antriebskraft zu erzeugen, und ein Nadelmodul aufweist, in dem eine Stechvorrichtung in Längsrichtung des Nadelmoduls verlagerbar angeordnet ist und welches an das Antriebsmodul lösbar gekoppelt ist, sodaß die Antriebskraft in die Stechvorrichtung eingeleitet ist, werden in verschiedenen Anwendungen genutzt, um eine Haut für das Einbringen eines Wirkstoffes in die Haut aufzustechen. Mit Hilfe der Antriebskraft wird die Stechvorrichtung repetierend vor und zurück bewegt. Bei dem einzubringenden Wirkstoff kann es sich beispielsweise um einen Farbstoff, einen kosmetischen Wirkstoff oder einen medizinischen Wirkstoff handeln. In Verbindung mit dem Farbstoff wird das Handgerät zum Beispiel als Tattoo- und Permanent-Make-up-Handgerät verwendet. Bei der medizinischen Anwendung können mit dem Handgerät Impfstoffe oder andere medizinisch wirksame Stoffe in die Haut eingebracht werden.

Aus dem Dokument DE 299 19 199 ist bekannt, bei derartigen Anordnungen zum wiederholten Aufstechen der Haut einen modulartigen Aufbau derart vorzusehen, daß das Handgerät mit einem Antriebsmodul und einem hieran lösbar zu befestigenden Nadelmodul zu bilden. Um eine korrekte Anwendung des Handgerätes sicherzustellen, ist es von besonderer Bedeutung, daß das Antriebsmodul, welches über die Antriebsvorrichtung verfügt, beispielsweise einen Elektromotor, mit dem richtigen Nadelmodul gekoppelt wird.

Das Dokument DE 299 23 931 U1 beschreibt ein Nadelstichgerät zum Tätowieren von Hautpartien. Das Nadelstichgerät umfaßt eine Antriebsmodul und ein Nadelmodul. Das Nadelmodul wird von einem Nadelträger sowie einem diesen Nadelträger aufnehmenden, hülsenförmigen Geräteabschnitt gebildet. In einer Ausführung sind an dem Nadelmodul warzenförmige Vorsprünge gebildet, die bei der Kopplung an das Antriebsmodul mit Nuten zusammenwirken.

Das Dokument US 2006/0129140 A1 beschreibt eine chirurgische Anordnung für die Augenheilkunde. Die Anordnung umfaßt einen Empfänger und eine Steuereinheit, welche in einem Steuergerät angeordnet sind, und ein chirurgisches Bauteil, beispielsweise einen Laser mit einem RFID-Chip. Der RFID-Chip überträgt an den Empfänger Daten, die dann von der Steuereinheit ausgewertet werden.

Im Dokument EP 1 118 919 A1 ist eine Werkzeugmaschine offenbart, bei der Lichtleitfasern vorgesehen sind. Die optischen Fasern dienen zum Erkennen eines Werkzeugs. Licht von Fasern wird zu optischen Lesefasern übertragen, wenn im Bereich zwischen den Fasern eine an dem Werkzeug gebildete Nut angeordnet ist.

Das Dokument DE 101 18 034 A1 betrifft einen wechselbaren Werkzeughalter, der auch als Wechselfutter bezeichnet wird. An der Werkzeugmaschine ist in einer Ausführungsform ein optischer Sensor vorgesehen, welcher Licht empfängt, dass an einer reflektierenden Marke an dem Werkzeughalter reflektiert wird. Das gleiche Messprinzip wird im Dokument FR 2,023,246 auf dem Gebiet der Werkzeugtechnik eingesetzt. Von einer Lichtquelle emittierte Strahlen werden reflektiert, sodass reflektierte Lichtstrahlen zu einem Detektor gelangen.

### Zusammenfassung der Erfindung

Aufgabe der Erfindung ist es deshalb, eine verbesserte Anordnung zum wiederholten Aufstechen einer Haut mit einem Handgerät und ein verbessertes Nadelmodul für die Anordnung zu schaffen, die eine ordnungsgemäße Nutzung von Antriebsmodul und Nadelmodul sicherstellen.

Diese Aufgabe wird erfindungsgemäß durch eine Anordnung zum wiederholten Aufstechen einer Haut nach dem unabhängigen Anspruch 1 und ein Nadelmodul für die Anordnung nach dem unabhängigen Anspruch 10 gelöst. Vorteilhafte Ausgestaltungen der Erfindung sind Gegenstand abhängiger Ansprüche.

Mit der Erfindung können dem Nadelmodul bei der Herstellung beliebige Kennungsinformationen beigegeben werden, die dann im Rahmen der Nutzung des Nadelmoduls in Verbindung mit dem Antriebsmodul auswertbar sind. Je nach Anwendungsfall kann das Nadelmodul mit Hilfe der Kennungsmittel individuell konfiguriert werden. Es ist so erleichtert, die Einhaltung von Sicherheitsvorschriften zu gewährleisten. Darüber hinaus ist es mit der Erfindung einfacher möglich, eine zweckentfremdende Nutzung des Nadelmoduls zu verhindern.

Die Auswertevorrichtung weist eine Sendeeinrichtung und eine Detektionseinrichtung auf, die konfiguriert ist, von der Sendeeinrichtung abgegebene Signale zu detektieren. Beispielsweise Wechselwirken die Signale mit den Kennungsmitteln, wodurch sich die Signale ändern. Die geänderten Signale werden dann von der Detektionseinrichtung detektiert.

Die Kennungsmittel umfassen Maskierungsmittel, die konfiguriert sind, die von der Sendeeinrichtung abgegebenen Signale zu maskieren. Die maskierten Signale werden anschließend von der Detektionseinrichtung detektiert. Auf diese Weise findet eine Auswertung der Kennungsmittel statt.

Die Maskierungsmittel umfassen eine Maske, die konfiguriert ist, die von der Sendeeinrichtung abgegebenen Signale einem dem Nadelmodul zugeordneten Code entsprechend zu maskieren. Die Maske ist mit einem Zylinder mit Aussparungen gebildet, zum Beispiel als eine Lochblende oder eine Lochmaske. Hierdurch ist erreicht, daß die Signale auf dem Signalweg zu der Detektionseinrichtung teilweise blockiert sind, wodurch eine Modulation der Signale erreicht ist, wobei die Modulation insbesondere von der Anzahl und / oder der Lage der Aussparungen oder dem Durchmesser der Löcher in der Lochblende abhängig ist. Auch kann vorgesehen sein, die Sendeeinrichtung an einer dem Nadelmodul zugewandten Fläche des Antriebsmoduls anzuordnen, beispielsweise im wesentlichen mittig auf der dem Nadelmodul zugewandten Fläche des Antriebsmoduls.

Weiterhin kann vorgesehen sein (nicht innerhalb des Schutzumfangs der Ansprüche), daß die Maske Signalleitmittel aufweist, die konfiguriert sind, die von der Sendeeinrichtung abgegebenen Signale der Detektionseinrichtung zuzuführen. Beispielsweise können die Signalleitmittel reflektierende Elemente aufweisen, welche die Signale reflektierten. Es kann insbesondere vorgesehen sein, daß eine schräge reflektierende Fläche an dem Nadelmodul gebildet ist, welche die Signale zu der Detektionseinrichtung reflektiert. Bevorzugterweise ist die Detektionseinrichtung an einem Vorsprung angeordnet. Wenn das Nadelmodul mit dem Antriebsmodul gekoppelt ist, reflektiert die schräge Fläche die von der Sendeeinrichtung abgegebenen Signale zu der Detektionseinrichtung. Beispielsweise werden in Abhängigkeit von einem Winkel der schrägen Fläche in Bezug auf eine Längsachse des Nadelmoduls die Signale zu unterschiedlichen Bereichen der Detektionseinrichtung reflektiert. Es kann aber auch vorgesehen sein, die schräge Fläche an unterschiedlichen Positionen an dem Nadelmodul anzuordnen. Somit ist eine Erkennung von unterschiedlichen Nadelmodulen dadurch ermöglicht, daß unterschiedlichen Bereichen, zu denen die Signale an der Detektionseinrichtung reflektiert werden, unterschiedlichen Nadelmodulen zugeordnet sind.

In einer Fortbildung, die nicht Teil der Erfindung ist, sind mehrere reflektierende Elemente benachbart zueinander an dem Nadelmodul angeordnet. In einer Ausgestaltung kann vorgesehen sein, daß zwischen zwei reflektierenden Elementen ein nicht reflektierender Bereich gebildet ist. Die Anordnung von reflektierenden Elementen und / oder nicht reflektierenden Bereichen bildet somit einen Erkennungsbereich. Insbesondere können die Sendeeinrichtung und die Detektionseinrichtung an einem Trägerbauteil gebildet sein, wobei das Trägerbauteil derart über das Antriebsmodul in Richtung des Nadelmoduls sich erstreckend an dem Antriebsmodul angeordnet ist, daß, wenn das Antriebsmodul und das Nadelmodul miteinander gekoppelt sind, das Trägerbauteil dem Erkennungsbereich gegenüber liegt. Beim Zusammenkoppeln des Nadelmoduls mit dem Antriebsmodul überstreicht somit das Trägerbauteil den Erkennungsbereich. Die von der Sendeeinrichtung abgegebenen Signale werden von dem Erkennungsbereich entsprechend reflektiert, wobei die reflektierten Signale von der Detektionseinrichtung detektiert werden. Insbesondere kann dann eine Erkennung des Nadelmoduls dadurch erreicht sein, daß die Anzahl von detektierten Signalen beispielsweise von einer Anzahl der reflektierenden Elemente abhängt.

In einer anderen Ausgestaltung kann vorgesehen sein, einen Referenzbereich an dem Nadelmodul zu bilden, wobei der Referenzbereich in einer Ausführung benachbart zueinander reflektierende Elemente umfaßt. Insbesondere kann der Referenzbereich auch nicht reflektierende Bereiche, welche beispielsweise zwischen zwei reflektierenden Elementen angeordnet sind, aufweisen. Der Anordnung von reflektierenden Elementen und / oder von nicht reflektierenden Bereichen ist bevorzugterweise einem Referenzcode zugeordnet. Insbesondere kann das Trägerbauteil derart gebildet sein, daß, wenn das Antriebsmodul mit dem Nadelmodul gekoppelt ist, das Trägerbauteil sowohl dem Erkennungsbereich als auch dem Referenzbereich gegenüber liegt. Beim Zusammenkoppeln des Nadelmoduls mit dem Antriebsmodul überstreicht somit das Trägerbauteil den Erkennungsbereich und den Referenzbereich. Die Detektionseinrichtung detektiert die von dem Erkennungsbereich und dem Referenzbereich reflektierten Signale, wobei die von dem Referenzbereich reflektierten Signale beispielsweise eine Referenz für die von dem Erkennungsbereich reflektierten Signale bilden.

Es kann ferner vorgesehen, daß die Sendeeinrichtung mit mindestens einer optische Signale abgebenden Lichtquelle gebildet ist, beispielsweise eine Leuchtdiode, eine organische Leuchtdiode, eine anorganische Leuchtdiode, eine polymere Leuchtdiode, eine hybride Leuchtdiode oder einen Laser. In einer Ausführung umfaßt die Detektionseinrichtung mindestens eine Photodiode. Insbesondere ist die Lichtquelle derart ausgebildet, daß die Lichtquelle diffuses Licht abgibt.

Eine bevorzugte Weiterbildung der Erfindung sieht vor, daß die Auswertevorrichtung konfiguriert ist, um die Kennungsmittel automatisch auszuwerten, wenn das Nadelmodul an das Antriebsmodul gekoppelt ist.

Bei einer zweckmäßigen Ausgestaltung der Erfindung kann vorgesehen sein, daß die Kennungsmittel zumindest eine der folgenden, von der Auswertevorrichtung auswertbaren und dem Nadelmodul zuordenbaren Informationen liefernd gebildet sind: Seriennummer, für das Nadelmodul nutzbare(s) Antriebsmodul(e), Nutzungsdauer des Nadelmoduls, Art der Stechvorrichtung und erlaubte Betriebsparameter wie Stechfrequenz und Betriebsarten.

Eine vorteilhafte Ausführungsform der Erfindung sieht vor, daß eine Steuervorrichtung gebildet ist, die konfiguriert ist, um einen Betrieb des Handgerätes zu steuern.

Eine bevorzugte Fortbildung der Erfindung sieht vor, daß die Steuervorrichtung in zumindest einem der folgenden Geräteteile implementiert ist: Handgerät und ein an das Handgerät gekoppeltes Steuergerät. Das Steuergerät wird in Verbindung mit Handgeräten zum Hautaufstechen üblicherweise dazu genutzt, Betriebsparameter zu regeln und eine Spannungsversorgung zur Verfügung zu stellen. Das Steuergerät selbst verfügt häufig über eine Anzeigeeinrichtung, Eingabemittel wie Drehknöpfe oder Tastenfelder.

Eine bevorzugte Weiterbildung der Erfindung sieht vor, daß die Auswertevorrichtung oder wahlweise die Steuervorrichtung konfiguriert sind, um den Betrieb des Handgerätes zu unterbinden, wenn beim Auswerten der Kennungsmittel festgestellt wird, daß das Nadelmodul für eine Nutzung mit dem Antriebsmodul nicht zugelassen ist. Wenn bei der Auswertung der Kennungsmittel festgestellt wird, daß das Nadelmodul für eine Nutzung mit dem Antriebsmodul nicht zugelassen ist, beispielsweise anhand der ausgewerteten Seriennummer, verhindern die Auswertevorrichtung oder wahlweise die Steuervorrichtung eine Nutzung des Handgerätes, was beispielsweise dadurch erreicht wird, daß eine Spannungsversorgung der Antriebsvorrichtung unterbrochen wird.

Bei einer zweckmäßigen Ausgestaltung der Erfindung kann vorgesehen sein, daß die Auswertevorrichtung oder wahlweise die Steuervorrichtung konfiguriert sind, um den Betrieb des Handgerätes der mindestens einen von der Auswertevorrichtung auswertbaren und dem Nadelmodul zuordenbaren Information entsprechend zu steuern. Eine Steuerung der auswertbaren Informationen entsprechend bedeutet beispielsweise eine Beschränkung des Betriebs des Handgerätes auf vorgegebene Betriebsarten, die zum Beispiel durch eine bestimmte Repetierfrequenz (Stechfrequenz) oder einen Frequenzbereich für die Stechvorrichtung oder eine Einstechtiefe charakterisiert sind, nämlich Parameter, die für den Betrieb des Handgerätes durch den Benutzer einstellbar sind, bevorzugt mittels des Steuergerätes.

Eine vorteilhafte Ausführungsform der Erfindung sieht vor, daß das Handgerät als eines der folgenden Handgeräte ausgeführt ist: Permanent-Make-up- und Tattoo-Handgerät, Wirkstoffabgabe-Handgerät und Hautimpf-Handgerät.

Eine bevorzugte Fortbildung der Erfindung sieht vor, daß das Nadelmodul ein sterilisiertes Einweg-Nadelmodul ist. Unabhängig von der Ausführung als Einwegmodul kann das Nadelmodul so gestaltet sein, dass es als Gesamtmodul oder in Form einzelner Elemente, beispielsweise als Gehäuse und Nadel oder Nadelsystem, an dem Antriebsmodul montierbar und von diesem Lösbar ist.

### Beschreibung bevorzugter Ausführungsbeispiele der Erfindung

Die Erfindung wird im folgenden anhand von bevorzugten Ausführungsbeispielen unter Bezugnahme auf Figuren einer Zeichnung näher erläutert. Hierbei zeigen:
- Fig. 1: schematisch eine Anordnung zum wiederholten Aufstechen einer Haut,
- Fig. 2: schematisch ein Handgerät,
- Fig. 3a: schematisch ein Handgerät in der Ausgestaltung in der Fig. 2, wobei das Na- delmodul und das Antriebsmodul miteinander gekoppelt sind,
- Fig. 3b: schematisch eine vergrößerte Teilansicht des Handgeräts aus Fig. 3a,
- Fig. 4a: schematisch ein weiteres Handgerät,
- Fig. 4b: schematisch das weitere Handgerät aus Fig. 4a in einer Seitenansicht,
- Fig. 4c: schematisch das weitere Handgerät aus Fig. 4a, aber mit einem anderen Na- delmodul als in Fig. 4b,
- Fig. 5a: schematisch ein anderes Handgerät,
- Fig. 5b: schematisch das Handgerät aus Fig. 5a in einer anderen Ansicht und
- Fig. 5c: schematisch das Handgerät aus Fig. 5a in einer weiteren Ansicht.

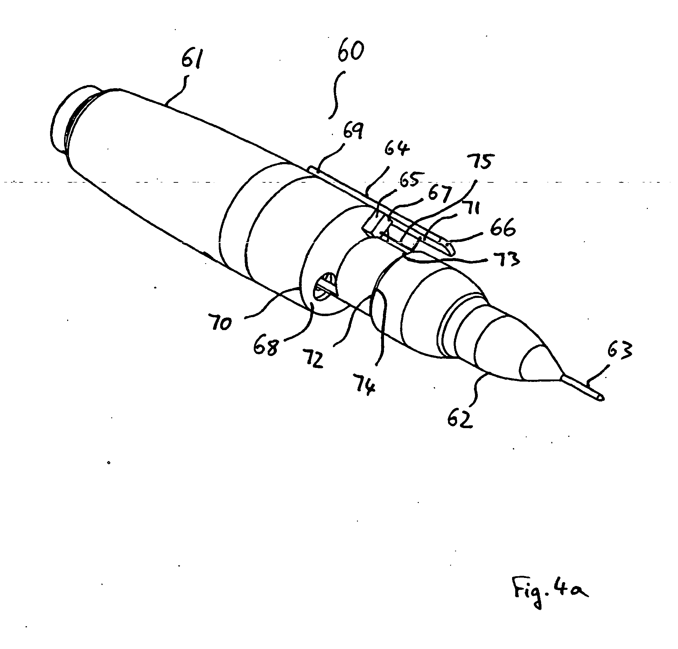

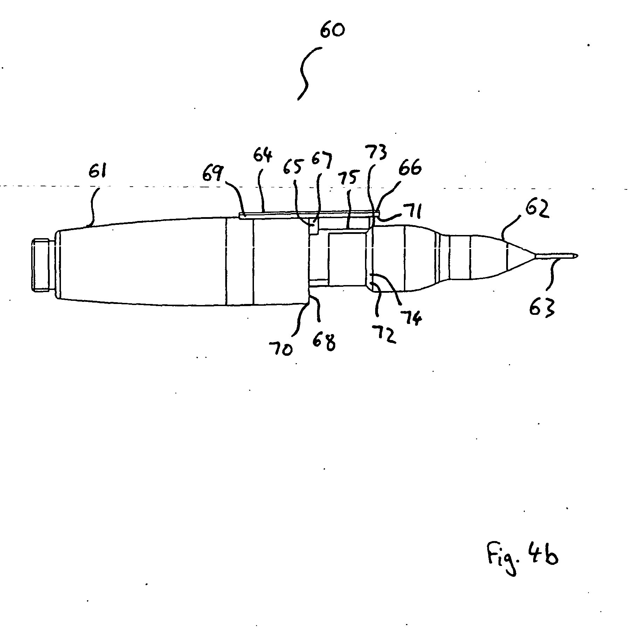

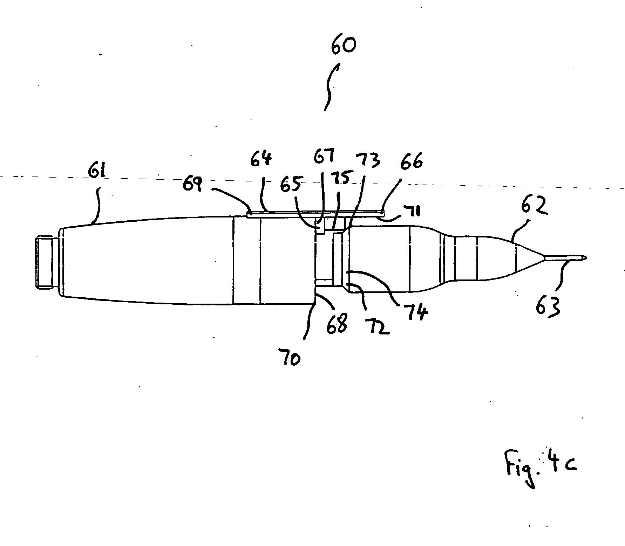

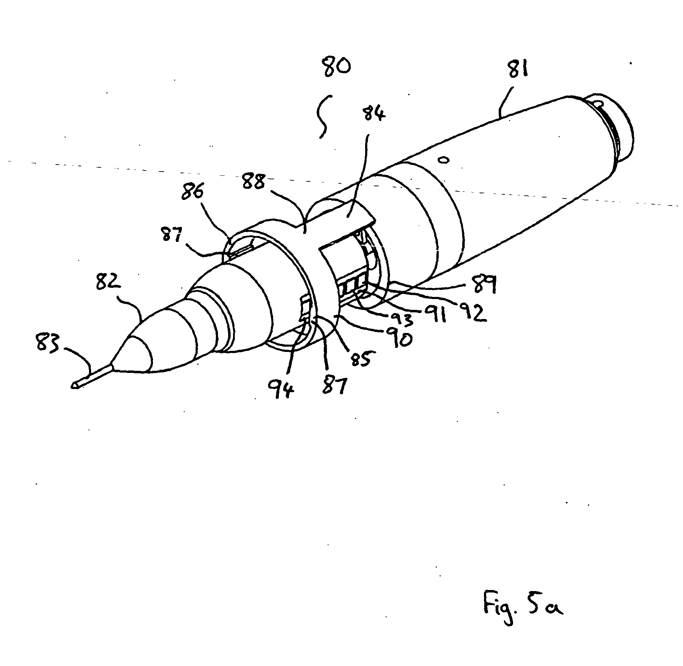

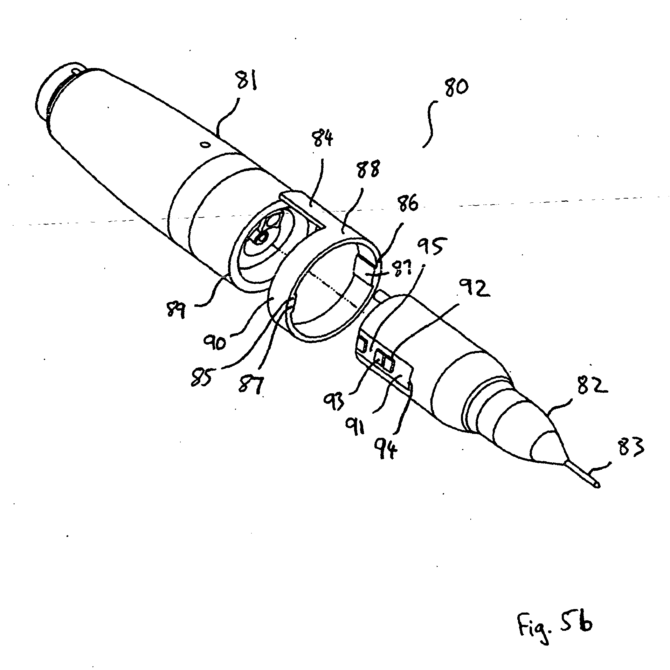

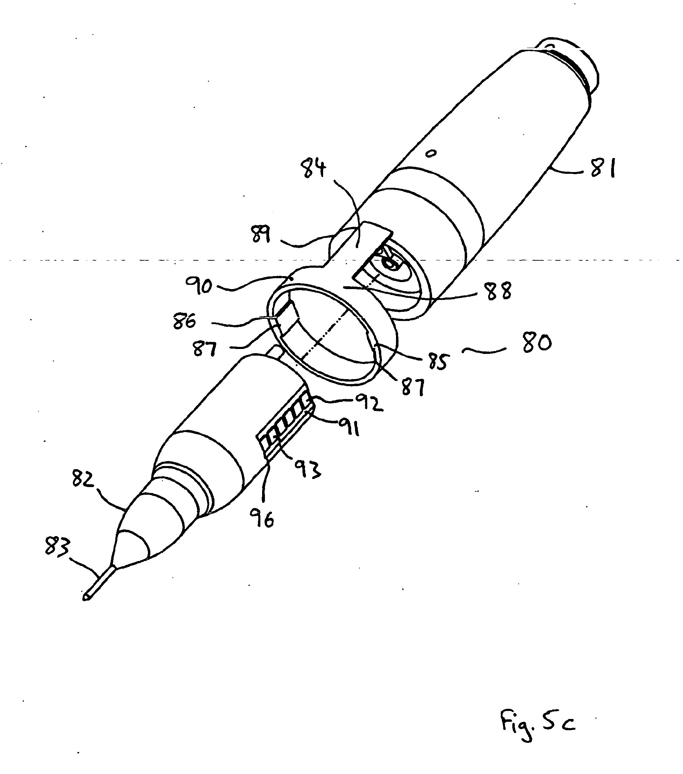

Die Figuren 4a, 4b, 4c, 5a, 5b, und 5c stellen die Erfindung nicht dar.

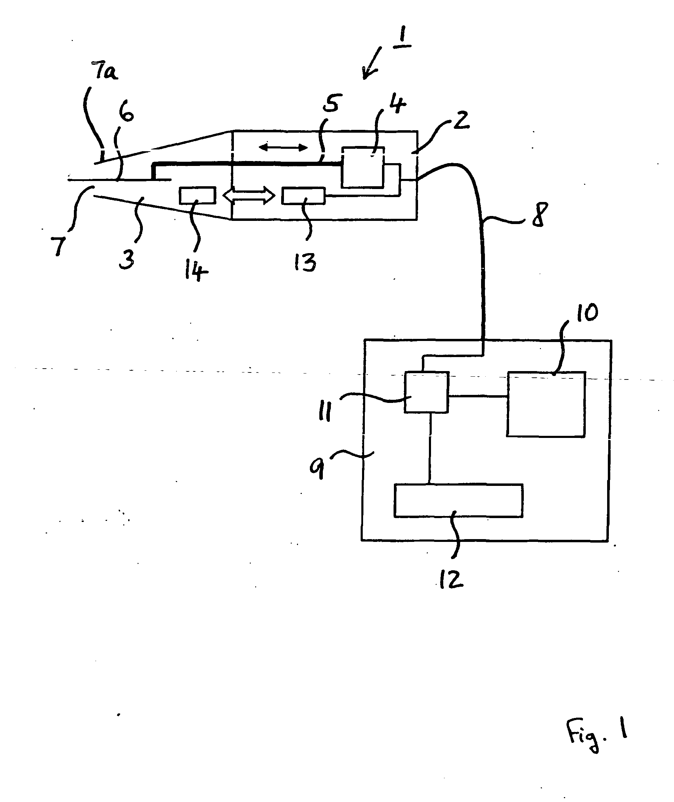

Fig. 1 zeigt schematisch eine Anordnung mit einem Handgerät 1 zum wiederholten Aufstechen einer Haut. Das Handgerät 1 umfaßt ein Antriebsmodul 2 sowie ein hieran lösbar gekoppeltes Nadelmodul 3. In dem Antriebsmodul 2 ist ein Elektromotor 4 als Antriebsvorrichtung vorgesehen, der über einen Kopplungsmechanismus 5, welcher seinerseits bevorzugt einen Taumelscheibenmechanismus umfaßt, an eine Nadel 6 koppelt. Die Nadel 6 bildet eine Stechvorrichtung zum Hautaufstechen, welche in anderen Ausführungen auch von einem System mehrerer Nadeln gebildet ist. Mit Hilfe des Elektromotors 4 und des Kopplungsmechanismus 5 wird eine repetierende Antriebsbewegung erzeugt, die über den Kopplungsmechanismus 5 auf die Nadel 6 eingeleitet wird, so daß die Nadel 6 durch eine Öffnung 7 des Nadelmoduls 3 hin und her bewegt wird. Üblicherweise ist die Nadel 6 mittels eines Nadelschaftes (nicht dargestellt) an den Kopplungsmechanismus 5 gekoppelt. Mit an die Öffnung 7 grenzenden Gehäuseabschnitten 7a des Nadelmoduls 3 ist eine Nadeldüse gebildet.

Das Nadelmodul 3 ist lösbar mit dem Antriebsmodul 2 verbunden, beispielsweise mittels einer Schraub-, einer Steck- oder einer Klemmverbindung. Auf diese Weise ist es ermöglicht, daß vorzugsweise als Einwegmodul ausgeführte Nadelmodul 3 nach einer Nutzung von dem Antriebsmodul 2 zu lösen und auszutauschen. Hierbei ist das Nadelmodul 3 so ausgeführt, daß es als Gesamtmodul von dem Antriebsmodul 2 gelöst werden kann. Das Nadelmodul 3 wird vorzugsweise als sterilisiertes Bauteil in einer geeigneten Verpackung zur Verfügung gestellt.

Über eine Verbindungsleitung 8 ist das Handgerät 1 mit einem Steuergerät 9 verbunden. Mit Hilfe der Verbindungsleitung 8 erfolgt insbesondere der Anschluß des Elektromotors 4 an eine Spannungsversorgung (nicht dargestellt). Darüber hinaus umfaßt die Verbindungsleitung 8 in der dargestellten Ausführungsform ein oder mehrere Leitungen zur Übertragung elektronischer Daten. In alternativen Ausgestaltungen kann das Handgerät 1 auch über eine kabellose Datenverbindung mit dem Steuergerät 9 verbunden sein. Eine Spannungsversorgung des Handgerätes 1 kann in einer Ausgestaltung mit Hilfe eines in das Handgerät 1 integrierten Akkus ausgeführt sein.

In dem Steuergerät 9 sind eine Anzeigenvorrichtung 10, eine Steuervorrichtung 11 sowie eine Bedienvorrichtung 12 gebildet. Weitere Bauteile oder Baugruppen können in dem Steuergerät 9 vorgesehen sein, werden hier aber zur Vereinfachung der Darstellung weggelassen. Mit Hilfe der Bedienvorrichtung 12 kann ein Benutzer Vorgaben für den Betrieb des Handgerätes 1 eingeben, beispielsweise über ein Tastenfeld oder mit Hilfe von Drehknöpfen. Über die Anzeigevorrichtung 11 werden Informationen zum Betrieb des Handgerätes 1 angezeigt, beispielsweise eine genutzte Betriebsart, eine gewählte Stechfrequenz oder dergleichen. Auch die Anzeige von Informationen betreffend das aktuell verwendete Nadelmodul 3 kann vorgesehen sein. Die Steuervorrichtung 10 dient insbesondere zum Steuern des Betriebs des Handgerätes 1 aber auch zur Koordination der Anzeige auf der Anzeigevorrichtung 11.

Gemäß der schematischen Darstellung in der Fig. 1 ist in dem Antriebsmodul 2 eine Auswertevorrichtung 13 gebildet, die genutzt wird, um Kennungsmittel 14 an dem Nadelmodul 3 auszuwerten. Die Kennungsmittel 14 und die Auswertevorrichtung 13 können mechanisch, optisch, kapazitiv oder induktiv miteinander gekoppelt sein. Beim Ankoppeln des Nadelmoduls 3 werden die Kennungsmittel 14 automatisch oder vom Benutzer initiiert, beispielsweise mittels Bedienung eines Tasters, von der Auswertevorrichtung 13 ausgewertet. Von der Auswertevorrichtung 13 werden dann bei der dargestellten Ausführungsform Datensignale an die Steuervorrichtung 10 übermittelt, die Informationen anzeigen, welche die Auswertevorrichtung 13 aus den Kennungsmitteln 14 abgeleitet hat. Beispielsweise kann es sich hierbei um ein Datensignal handeln, welches anzeigt, daß das Nadelmodul 3 passend zu dem Antriebsmodul 2 ist. Ein solches Signal kann zum Beispiel mit Hilfe eines an dem Antriebsmodul 2 angebrachten Tastschalters erzeugt werden, welcher bedient wird, wenn das Nadelmodul 3 an das Antriebsmodul 2 gekoppelt wird, indem beispielsweise ein Vorsprung (nicht dargestellt) an dem Nadelmodul 3 beim Koppeln auf den Tastschalter drückt, welcher in die Auswertevorrichtung 13 integriert ist.

In Abhängigkeit von den von der Auswertevorrichtung 13 erhaltenen Datensignalen steuert die Steuervorrichtung 10 dann den Betrieb des Handgerätes 1. Beispielsweise kann der Betrieb des Handgerätes 1 freigegeben oder gesperrt werden, je nach dem ob die Datensignale das Koppeln eines korrekten Nadelmoduls oder eines nicht mit dem Antriebsmodul 2 nicht zugelassenen Nadelmoduls anzeigen. Des weiteren können die mittels der Kennungsmittel 14 zur Verfügung gestellten Informationen zugelassene Betriebsarten des Nadelmoduls 3 oder Nutzungsbeschränkungen des Nadelmoduls 3 umfassen, beispielsweise hinsichtlich einer Nutzungsdauer. Die Steuereinrichtung 10 ist konfiguriert, um die von der Auswertevorrichtung 13 übersandten Datensignale dann entsprechend bei der Steuerung des Betriebs des Handgerätes 1 zu berücksichtigen.

Alternativ zu der Ausführungsform in Fig. 1 kann die Steuervorrichtung 10 zumindest teilweise in das Handgerät 1 integriert sein, beispielsweise in die Auswertevorrichtung 13. Zu diesem Zweck ist zum Beispiel in Mikroprozessor (nicht dargestellt) in das Handgerät 1 integriert, nämlich in das Antriebsmodul 2. Die Nutzung der aus den Kennungsmitteln 14 abgeleiteten Informationen für die Steuerung des Handgerätes 1 kann dann zumindest teilweise in dem Handgerät 1 selbst erfolgen.

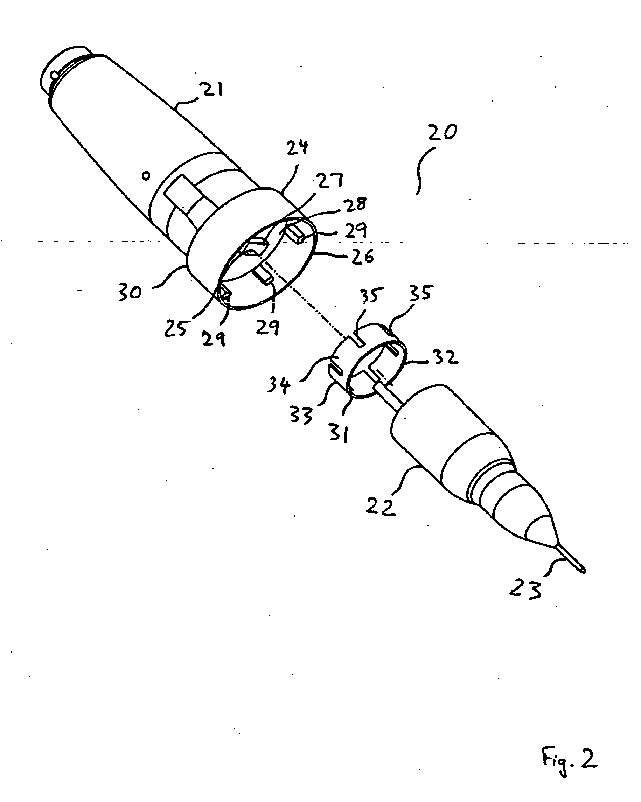

Fig. 2 zeigt ein Handgerät 20 mit einem Antriebsmodul 21 und einem Nadelmodul 22. An dem Nadelmodul 22 ist eine Nadel 23 angeordnet. Die Nadel 23 bildet eine Stechvorrichtung zum Hautaufstechen, welche in anderen Ausführungen von einem System mehrerer Nadeln gebildet sein kann.

An dem Antriebsmodul 21 ist eine Auswertevorrichtung 24 mit einer Sendeeinrichtung 25 und einer Detektionseinrichtung 26. Die Sendeeinrichtung 25 ist als eine Lichtquelle 27, insbesondere eine Leuchtdiode, ausgeführt. Beispielsweise kann die Sendeeinrichtung 25 auch ein Laser sein. Die Lichtquelle 27 ist im wesentlichen mittig auf einer dem Nadelmodul 22 zugewandten Fläche 28 des Antriebsmoduls 21 angeordnet. Die Detektionseinrichtung 26 umfaßt mehrere Lichtsensoren 29, welche auf einer inneren Fläche eines Sensorrings 30 angeordnet sind. Der Sensorring 30 verläuft um die dem Nadelmodul 22 zugewandte Fläche 28 des Antriebsmoduls 21.

An dem Nadelmodul 22 sind Kennungsmittel 31 gebildet. Die Kennungsmittel 31 umfassen Maskierungsmittel 32 zum Maskieren der von der Lichtquelle 27 abgegebenen Lichtsignale. In dieser Ausführungsform sind die Maskierungsmittel 32 als eine Maske 33 mit einem Zylinder 34 gebildet, welcher Aussparungen 35 aufweist.

Wenn das Nadelmodul 22 und das Antriebsmodul 21 miteinander gekoppelt sind, ist der Zylinder 34 in einem inneren Bereich des Sensorrings 30 angeordnet. Die Lichtquelle 27 ist dann im wesentlichen mittig in der Maske 33 angeordnet. Der Zylinder 34 blockiert die Lichtsignale, und die Aussparungen 35 lassen die Lichtsignale zu den Lichtsensoren 29 durch. Hierdurch maskiert die Maske 33 die von der Lichtquelle 27 ausgesandten Lichtsignale. Beispielsweise kann die Anzahl und / oder die Lage der Aussparungen 35 eine dem Nadelmodul 22 zugeordnete Information entsprechen. Die Information kann beispielsweise die Seriennummer oder erlaubte Betriebsparameter wie Stechfrequenz und Betriebsarten umfassen. Somit detektieren die Lichtsensoren 29 eine gleiche Anzahl an Lichtsignalen wie die Anzahl an Aussparungen 35. Hierdurch ist erreicht, daß die Information an das Antriebsmodul 21 übermittelt wird.

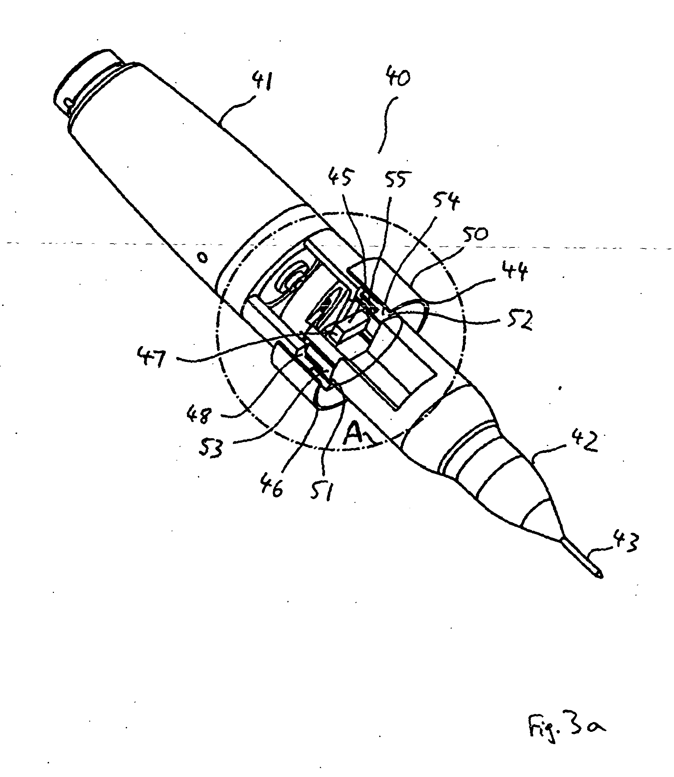

Fig. 3a zeigt schematisch ein Handgerät 40 mit einem Antriebsmodul 41 und einem an das Antriebsmodul 41 gekoppeltes Nadelmodul 42 in der Ausgestaltung in der Fig. 2. An dem Nadelmodul 42 ist eine Nadel 43 gebildet. An dem Antriebsmodul 41 ist eine Auswertevorrichtung 44 gebildet, welche eine Sendeeinrichtung 45 und eine Detektionseinrichtung 46 umfaßt. Die Sendeeinrichtung 45 ist als eine Lichtquelle 47 ausgeführt, wobei die Lichtquelle 47 an einer dem Nadelmodul 42 zugewandten Fläche 48 des Antriebsmoduls 41 angeordnet ist. Die Detektionseinrichtung 46 umfaßt Lichtsensoren (nicht gezeigt), welche auf einer inneren Fläche eines Sensorrings 50 angeordnet sind. Der Sensorring 50 verläuft um die dem Nadelmodul 42 zugewandte Fläche 48.

An dem Nadelmodul 42 sind Kennungsmittel 51 gebildet, wobei die Kennungsmittel 51 Maskierungsmittel 52 zum Maskieren der von der Lichtquelle 47 abgegebenen Lichtsignale aufweist. Die Maskierungsmittel 52 sind als eine Maske 53 mit einem Zylinder 54 gebildet, welcher Aussparungen 55 aufweist. Die Maske 53 maskiert in der gleichen Art und Weise wie die Maske 33 in der Fig. 2 die von der Lichtquelle 57 ausgesandten Lichtsignale.

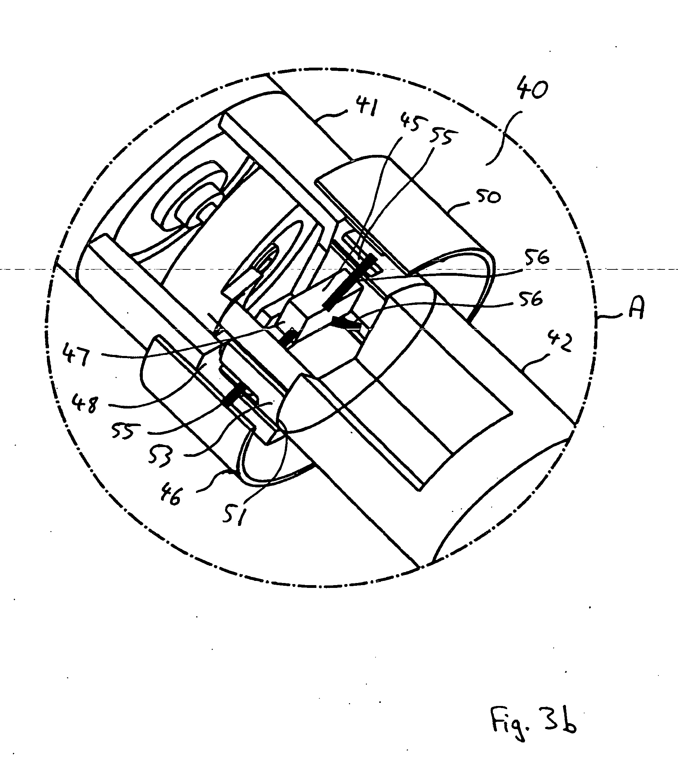

Fig. 3b zeigt schematisch eine vergrößerte Teilansicht des mit "A" bezeichneten Bereiches aus Fig. 3a. In Fig. 3b werden für gleiche Elemente die gleichen Bezugszeichen wie in Fig. 3a verwendet. Weiterhin zeigt Fig. 3b zusätzlich Lichtwege 56 für die von der Lichtquelle 47 ausgesandten Lichtsignale. Deutlich erkennbar ist, daß die Aussparungen 55 die Lichtsignale zu den Lichtsensoren durchlassen.

Fig. 4a, 4b und 4c zeigen schematisch jeweils ein Handgerät 60 mit einem Antriebsmodul 61 und einem Nadelmodul 62. An dem Nadelmodul 62 ist eine Nadel 63 angeordnet.

An dem Antriebsmodul 61 ist eine Auswertevorrichtung 64 gebildet. Die Auswertevorrichtung 64 umfaßt eine Sendeeinrichtung 65 und eine Detektionseinrichtung 66. Die Sendeeinrichtung 65 ist als eine Lichtquelle 67 ausgeführt. Die Lichtquelle 67 ist in einem äußeren Bereich einer dem Nadelmodul 62 zugewandten Fläche 68 angeordnet. Die Detektionseinrichtung 66 ist an einem Trägerbauteil 69 gebildet, wobei das Trägerbauteil 69 sich in Richtung des Nadelmoduls 62 über das Antriebsmodul 61 erstreckend an dem Antriebsmodul 61 angeordnet ist. Bevorzugterweise kann die Lichtquelle 67 in Kontakt mit dem Trägerbauteil 69 auf der dem Nadelmodul 62 zugewandten Fläche 68 angeordnet sein, um das Trägerbauteil 69 zusätzlich zu stützen. An dem Trägerbauteil 69 sind auf einer der Fläche 68 zugewandten Seite mehrere Lichtsensoren angeordnet, wodurch eine Lichtsensorreihe 71 gebildet ist.

An dem Nadelmodul 62 sind Kennungsmittel 72 gebildet. Die Kennungsmittel umfassen Signalleitmittel 73 zum Leiten der von der Lichtquelle 67 ausgesandten Lichtsignale 75. Die Signalleitmittel 73 sind als eine reflektierende Fläche 74 gebildet. Die reflektierende Fläche 74 ist unter einem Winkel in Bezug auf eine longitudinale Achse des Handgeräts 60 an dem Nadelmodul 62 angeordnet, so daß die Lichtsignale 75 auf die Sensorreihe 71 reflektiert sind, wenn das Nadelmodul 62 mit dem Antriebsmodul 61 gekoppelt ist.

Die reflektierende Fläche 74 in der Fig. 4b ist in einem anderen Bereich des Nadelmoduls 62 angeordnet als die reflektierende Fläche 74 in der Fig. 4c. Hierdurch ist erreicht, daß die Lichtsignale 74 auf unterschiedliche Bereiche der Sensorreihe 71 reflektiert werden, wenn das Antriebsmodul 61 mit dem Nadelmodul 61 gekoppelt ist. Beispielsweise kann eine Information einer Positionierung der reflektierenden Fläche 74 an dem Nadelmodul 62 zugeordnet sein. Die Information kann beispielsweise eine Seriennummer oder eine Betriebsart des Nadelmoduls 62 umfassen. Die Detektionseinrichtung 66 detektiert die entsprechende Position der von der Fläche 74 auf die Sensorreihe 71 reflektierten Lichtsignale 74, wodurch die Information an das Antriebsmodul 61 übermittel wird.

Fig. 5a, 5b und 5c zeigen schematisch jeweils ein Handgerät 80 mit einem Antriebsmodul 81 und einem Nadelmodul 82. An dem Nadelmodul 82 ist eine Nadel 83 angeordnet.

An dem Antriebsmodul 81 ist eine Auswertevorrichtung 84 gebildet. Die Auswertevorrichtung 84 umfaßt eine Sendeeinrichtung 85 und eine Detektionseinrichtung 86. Die Sendeeinrichtung 85 umfaßt mehrere Lichtquellen (nicht gezeigt). Die Detektionseinrichtung 86 umfaßt mehrere Sensoren (nicht gezeigt). Die mehreren Lichtquellen und die mehreren Sensoren sind benachbart zueinander in mehrere Bereiche 87 angeordnet. Die mehreren Bereiche 87 sind an einem Trägerbauteil 88 gebildet, wobei das Trägerbauteil 88 sich über ein dem Nadelmodul 82 zugewandtes Ende 89 des Antriebsmoduls 81 hinaus erstreckend an dem Antriebsmodul 81 angeordnet ist. Das Trägerbauteil 88 weist an einem dem Antriebsmodul 81 abgewandten Ende eine Ringform auf, wobei die mehreren Bereiche 87 auf einer inneren Fläche der Ringform angeordnet ist, wodurch ein Sensorring 90 gebildet ist.

An dem Nadelmodul 82 sind Kennungsmittel 91 gebildet. Die Kennungsmittel 91 umfassen Signalleitmittel 92 zum Leiten der von den mehreren Lichtquellen abgegebenen Lichtsignale. Die Signalleitmittel 92 sind als reflektierende Elemente 93 gebildet. Die reflektierenden Elemente 93 sind in einem Erkennungsbereich 94 benachbart zueinander angeordnet. Es kann vorgesehen sein, daß zwischen zwei reflektierenden Elementen 93 ein nicht reflektierender Bereich 95, wie in Fig. 5b gezeigt, gebildet ist.

Wenn das Nadelmodul 82 und das Antriebsmodul 81 miteinander gekoppelt werden, so überstreicht die Sendeeinrichtung 85 den Erkennungsbereich 94 und die Lichtsignale werden von den reflektierenden Elementen 93 reflektiert und von den mehreren Sensoren detektiert. Beispielsweise kann der Anzahl der reflektierenden Elemente 93 eine dem Nadelmodul 82 zugeordnete Information zugeordnet sein. Die Information wird dadurch an das Antriebsmodul 81 übertragen, indem die Anzahl der reflektierten Lichtsignale mittels der Sensoren detektiert sind. Beispielsweise kann ein detektiertes reflektiertes Lichtsignal der binären Eins entsprechen. Die binäre Null entspricht dann dem Fall, wenn kein reflektiertes Lichtsignal detektiert ist, also wenn die mehreren Lichtquellen über den nicht reflektierenden Bereich 95 in dem Erkennungsbereich 94 streichen.

Fig. 5c zeigt zusätzlich zu dem Erkennungsbereich (nicht gezeigt) noch einen an dem Nadelmodul 82 gebildeten Referenzbereich 96. Der Referenzbereich 96 umfaßt benachbart zueinander angeordnete reflektierende Elemente 93. In einem anderen Beispiel können auch nicht reflektierende Bereiche zwischen den reflektierenden Elementen 93 gebildet sein. Beispielsweise kann die Anzahl der reflektierenden Elemente 93 und / oder der nicht reflektierenden Bereiche 95 in dem Referenzbereich 96 bekannt sein, wodurch eine Zuordnung der detektierten reflektierten Lichtsignale zu der binären Eins vereinfacht ermöglicht ist.

## Patentansprüche

1. Anordnung zum wiederholten Aufstechen einer Haut, mit:
- einem Handgerät (20; 40) mit einem Antriebsmodul (21; 41) und einem Nadelmodul (22; 42), wobei das Nadelmodul (22; 42), lösbar an das Antriebsmodul (21; 41) gekoppelt ist,
- einer Antriebsvorrichtung (4), die in dem Antriebsmodul gebildet und konfiguriert ist, eine Antriebskraft zu erzeugen,
- einer Stechvorrichtung (23; 43), die in dem Nadelmodul gebildet ist, die in Längsrichtung des Nadelmoduls (22; 42) verlagerbar angeordnet ist und in die die Antriebskraft der Antriebsvorrichtung (4) eingeleitet ist, und
- Kennungsmitteln (31; 51) an dem Nadelmodul (22; 42),
**dadurch gekennzeichnet, daß**
- eine Auswertevorrichtung (24; 44) vorgesehen ist, die den Kennungsmitteln (31 ;51) zugeordnet ist, die von dem Antriebsmodul (21; 41) umfaßt ist und mit der die Kennungsmittel (31; 51) mit Hilfe einer optischen Auswertung auswertbar sind,
- eine Sendeeinrichtung (25; 45) und eine Detektionseinrichtung (26; 46) gebildet sind, die von der Auswertevorrichtung (24; 44) umfaßt sind, wobei die Detektionseinrichtung (26; 46) konfiguriert ist, von der Sendeeinrichtung (25; 45) abgegebene Signale zu detektieren,
- die Detektionseinrichtung (26; 46) mit mehreren Lichtsensoren (29) gebildet ist, die auf einer inneren Fläche eines Sensorrings (30; 50) um eine Lichtquelle (27; 47) angeordnet sind,
- die Kennungsmittel (31; 51) Maskierungsmittel (32; 52) umfassen, die konfiguriert sind, die von der Sendeeinrichtung (25; 45) abgegebenen Signale zu maskieren, und
- die Maskierungsmittel (32; 52) eine Maske (33; 53) umfassen, die mittels eines Zylinders (34; 54) mit Aussparungen (35; 55) konfiguriert ist, die von der Sendeeinrichtung (45; 65) abgegebenen Signale einem dem Nadelmodul (22; 42) zugeordneten Code entsprechend zu maskieren, wobei die Aussparungen (35; 55) an dem Zylinder (34; 54) gebildet sind, von der Lichtquelle (27; 47) ausgesandte Lichtsignale zu den mehreren Lichtsensoren (29) auf der inneren Fläche des Sensorrings (30; 50) durchzulassen.

2. Anordnung nach Anspruch 1, **dadurch gekennzeichnet, daß** die Auswertevorrichtung (24; 44) konfiguriert ist, die Kennungsmittel (31; 51) automatisch auszuwerten, wenn das Nadelmodul (22; 42) an das Antriebsmodul (21; 41) gekoppelt ist.

3. Anordnung nach mindestens einem der vorangehenden Ansprüche, **dadurch gekennzeichnet, daß** die Kennungsmittel (31; 51) zumindest eine der folgenden, von der Auswertevorrichtung (24; 44) auswertbaren und dem Nadelmodul (22; 42) zuordenbaren Informationen liefernd gebildet sind: Seriennummer, für das Nadelmodul (22; 42) nutzbare(s) Antriebsmodul(e) (21; 41), Nutzungsdauer des Nadelmoduls (22; 42), Art der Stechvorrichtung (23; 43) und erlaubte Betriebsparameter wie Stechfrequenz und Betriebsarten.

4. Anordnung nach mindestens einem der vorangehenden Ansprüche, **dadurch gekennzeichnet, daß** eine Steuervorrichtung (10) gebildet ist, die konfiguriert ist, einen Betrieb des Handgerätes (20; 40) zu steuern.

5. Anordnung nach Anspruch 4, **dadurch gekennzeichnet, daß** die Steuervorrichtung (10) in zumindest einem der folgenden Geräteteile implementiert ist: Handgerät (20; 40) und ein an das Handgerät (20; 40) gekoppeltes Steuergerät (9).

6. Anordnung nach mindestens einem der vorangehenden Ansprüche, **dadurch gekennzeichnet, daß** die Auswertevorrichtung (24; 44) oder wahlweise die Steuervorrichtung (10) konfiguriert sind, den Betrieb des Handgerätes (20; 40) zu unterbinden, wenn beim Auswerten der Kennungsmittel (31; 51) festgestellt wird, daß das Nadelmodul (22; 42) für eine Nutzung mit dem Antriebsmodul (21; 41) nicht zugelassen ist.

7. Anordnung nach Anspruch 3 oder mindestens einem der Ansprüche 4 bis 6, soweit auf Anspruch 3 rückbezogen, **dadurch gekennzeichnet, daß** die Auswertevorrichtung (24; 44) oder wahlweise die Steuervorrichtung (10) konfiguriert sind, den Betrieb des Handgerätes (20; 40) der mindestens einen von der Auswertevorrichtung (24; 44) auswertbaren und dem Nadelmodul (22; 42) zuordenbaren Information entsprechend zu steuern.

8. Anordnung nach mindestens einem der vorangehenden Ansprüche, **dadurch gekennzeichnet, daß** das Handgerät (20; 40) als eines der folgenden Handgeräte (20; 40) ausgeführt ist: Permanent-Make-up- und Tattoo-Handgerät, Wirkstoffabgabe-Handgerät und Hautimpf-Handgerät.

9. Anordnung nach mindestens einem der vorangehenden Ansprüche, **dadurch gekennzeichnet, daß** das Nadelmodul (22; 42) ein sterilisiertes Einweg-Nadelmodul (22; 42) ist.

10. Nadelmodul für eine Anordnung nach mindestens einem der vorangehenden Ansprüche, mit:
- einem Nadelmodul-Gehäuse,
- Kopplungsmitteln an dem Nadelmodul-Gehäuse, die konfiguriert sind, das Nadelmodul-Gehäuse an ein Antriebsmodul (21; 41) mit einer Antriebsvorrichtung (4) lösbar zu koppeln,
- einer Stechvorrichtung, welche in Längsrichtung verlagerbar in dem Nadelmodul-Gehäuse angeordnet ist, und
- Kennungsmitteln (31; 51), die mit einer den Kennungsmitteln (31; 51) zugeordneten Auswertevorrichtung (24; 44) optisch auswertbar sind,
wobei die Kennungsmittel (31; 51) Maskierungsmittel (32; 52) umfassen, die konfiguriert sind, die von einer Sendeeinrichtung (25; 45) abgegebenen Signale zu maskieren, und
wobei die Maskierungsmittel (32; 52) eine Maske (33; 53) umfassen, die mittels eines Zylinders (34; 54) mit Aussparungen (35; 55) konfiguriert ist, die von der Sendeeinrichtung (45; 65) abgegebenen Signale einem dem Nadelmodul (22; 42) zugeordneten Code entsprechend zu maskieren, wobei die Aussparungen (35; 55) an dem Zylinder (34; 54) gebildet sind, von einer Lichtquelle (27; 47) ausgesandte Lichtsignale zu mehreren Lichtsensoren (29) auf der inneren Fläche eines Sensorrings (30; 50) durchzulassen.

## Claims

1. An assembly for a repeated piercing of a skin, with:
- a hand-held device (20; 40) having a drive module (21; 41) and a needle module (22; 42), wherein the needle module (22; 42) is detachably coupled to the drive module (21; 41),
- a drive unit (4) that is formed in the drive module (21; 41) and is configured for the purpose of producing a driving force,
- a piercing apparatus (23; 43) that is formed in the needle module (22; 42) and is moveably arranged in a longitudinal direction of the needle module (22; 42), wherein the driving force of the drive unit (4) is conducted into said piercing apparatus (23; 43), and
- identification means (31; 51) at the needle module (22; 42),
**characterized in that**
- an evaluation apparatus (24; 44) is provided that is allocated to the identification means (31; 51) and is included in the drive module (21; 41), wherein the identification means (31; 51) can be evaluated by means of an optical evaluation by said evaluation apparatus (24; 44),
- a transmission device (25; 45), that is designed as a light source (27; 47), and a detection device (26; 46) are formed included in the evaluation apparatus (24; 44), wherein the detection device (26; 46) is configured for the purpose of detecting signals discharged from the transmission device (25; 45),
- the detection device (26; 46) is formed with a multiple of light sensors (29) that are arranged on an inner area of a sensor ring (30; 50) around the light source (27; 47),
- the identification means (31; 51) comprise masking means (32; 52) which are configured for the purpose of masking the signals discharged from the transmission device (25; 45), and
- the masking means (32; 52) comprise a mask (33; 53) that is configured for the purpose of masking the signals discharged by the transmission device (25; 45) according to a code allocated to the needle module (22; 42) by a cylinder (34; 54) with notches (35; 55), wherein the notches (35; 55) are formed in such a way at the cylinder (34; 54) that light signals discharged by the light source (27; 47) to the multiple light sensors (29) on the inner area of the sensor ring (30; 50) can pass through.

2. The assembly according to Claim 1, **characterized in that** the evaluation apparatus (24; 44) is configured for the purpose of automatically evaluating the identification means (31; 51) if the needle module (22; 42) is coupled to the drive module (31;41).

3. The assembly according to at least one of the preceding Claims, **characterized in that** the identification means (31; 51) are formed to supply at least one of the following information items that can be evaluated by the evaluation apparatus (24; 44) and that can be allocated to the needle module (22; 42): serial number, for the drive module(s) (21; 41) usable for the needle module (22; 42), usage duration of the needle module (22; 42), type of piercing apparatus (23; 43) and allowed operating parameters such as piercing frequency and operating modes.

4. The assembly according to at least one of the preceding Claims, **characterized in that** a control device (10) is formed which is configured to control an operation of the hand-held device (20; 40).

5. The assembly according to Claim 4, **characterized in that** the control device (10) is implemented in at least one of the following equipment components: hand-held device (20; 40) and a control unit (9) coupled to the hand-held device (20; 40).

6. The assembly according to at least one of the preceding Claims, **characterized in that** the evaluation apparatus (24; 44) or selectively the control device (10) are configured for the purpose of blocking the operation of the hand-held device (20; 40) if it is determined during the evaluation of the identification means (31; 51) that the needle module (22; 42) is not approved for usage with the drive module (21; 41).

7. The assembly according to Claim 3 or at least one of the Claims 4 to 6, insofar as referred to Claim 3, **characterized in that** the evaluation apparatus (24; 44) or selectively the control device (10) are configured for the purpose of controlling the operation of the hand-held (20; 40) device according to at least one information item that can be evaluated by the evaluation apparatus (24; 44) and that is allocated to the needle module (22; 42).

8. The assembly according to at least one of the preceding Claims, **characterized in that** the hand-held device (20; 40) is designed as one of the following hand-held devices (20; 40): permanent make-up and tattoo hand-held device, hand-held device for discharging active ingredients, and skin vaccination hand-held device.

9. The assembly according to at least one of the preceding Claims, **characterized in that** the needle module (22; 42) is a sterilised and disposable needle module (22; 42).

10. A needle module for an assembly according to at least one of the preceding Claims, with:
- a needle module casing,
- coupling means at the needle module casing that are configured for a detachable coupling of the needle module casing to a drive module (21; 41) with a drive unit (4),
- a piercing apparatus which is moveably arranged in a longitudinal direction in the needle module casing
- identification means (31; 51) which can be evaluated with an evaluation apparatus (24; 44) allocated to the identification means (31; 51) by means of an optical evaluation,
wherein the identification means (31; 51) comprise masking means (32; 52) which are configured for the purpose of masking signals discharged from the transmission device (25; 45) that is designed as a light source (27; 47), and
wherein the masking means (32; 52) comprise a mask (33; 53) that is configured for the purpose of masking the signals discharged by the transmission device (25; 45) according to a code allocated to the needle module (22; 42) by a cylinder (34; 54) with notches (35; 55), wherein the notches (35; 55) are formed in such a way at the cylinder (34; 54) that light signals discharged by the light source (27; 47) to the multiple light sensors (29) on the inner area of the sensor ring (30; 50) can pass through.

## Revendications

1. Arrangement pour le perçage répété d'une peau, avec :
- un appareil à main (20 ; 40) avec un module d'entraînement (21 ; 41) et un module à aiguille (22 ; 42), le module à aiguille (22 ; 42) étant couplé de manière amovible au module d'entraînement (21 ; 41),
- un dispositif d'entraînement (4) qui est formé dans le module d'entraînement et configuré de manière à générer une force motrice,
- un dispositif de perçage (23 ; 43) qui est formé dans le module à aiguille qui est disposé de manière décalable dans le sens longitudinal du module à aiguille (22 ; 42) et dans lequel est introduite la force motrice du dispositif d'entraînement (4), et
- des moyens d'identification (31 ; 51) sur le module à aiguille (22 ; 42),
**caractérisé en ce que**
- est prévu un dispositif d'exploitation (24 ; 44) qui est affecté aux moyens d'identification (31 ; 51) que comprend le module d'entraînement (21 ; 41) et avec lequel les moyens d'identification (31 ; 51) peuvent être exploités à l'aide d'une exploitation optique,
- sont formés un équipement émetteur (25 ; 45), réalisé comme source de lumière (27 ; 47), et un équipement de détection (26 ; 46) que comprend le dispositif d'exploitation (24 ; 44), étant donné que le dispositif d'exploitation (24 ; 44) est configuré de manière à détecter les signaux émis par l'équipement détecteur (25 ; 45),
- l'équipement de détection (26 ; 46) est formé avec plusieurs capteurs de lumière (29) qui sont disposés sur une surface intérieure d'un anneau à capteurs (30 ; 50) autour de la source de lumière (27 ; 47),
- les moyens d'identification (31 ; 51) comprennent des moyens de masquage (32 ; 52) qui sont configurés de manière à masquer les signaux émis par l'équipement émetteur (25 ; 45), et
- les moyens de masquage (32 ; 52) comprennent un masque (33 ; 53) qui est configuré au moyen d'un cylindre (34 ; 54) avec des évidements (35 ; 55) de manière à masquer conformément au code affecté au module à aiguille (22 ; 42) les signaux émis par l'équipement émetteur (45 ; 65), étant donné que les évidements (35 ; 55) sont formés sur le cylindre (34 ; 54) pour laisser passer les signaux lumineux émis par la source de lumière (27 ; 47) aux plusieurs capteurs de lumière (29) se trouvant sur la surface intérieure de l'anneau de capteurs (30 ; 50).

2. Arrangement selon la revendication 1, **caractérisé en ce que** le dispositif d'exploitation (24 ; 44) est configuré de manière à exploiter automatiquement les moyens d'identification (31 ; 51) lorsque le module à aiguille (22 ; 42) est couplé au module d'entraînement (21 ; 41).

3. Arrangement selon au moins l'une quelconque des revendications précédentes, **caractérisé en ce que** les moyens d'identification (31 ; 51) sont formés de manière à fournir au moins une des informations suivantes exploitables par le dispositif d'exploitation (24 ; 44) et pouvant être affectées au module à aiguille (22 ; 42) : numéro de série, module(s) d'entraînement (21 ; 41) utilisable(s) pour le module à aiguille (22 ; 42), durée d'utilisation du module à aiguille (22 ; 42), type de dispositif de perçage (23 ; 43) et paramètres de service autorisés comme fréquence de perçage et modes de fonctionnement.

4. Arrangement selon au moins l'une quelconque des revendications précédentes, **caractérisé en ce qu'**est formé un dispositif de commande (10) qui est configuré de manière à commander un fonctionnement de l'appareil à main (20 ; 40).

5. Arrangement selon la revendication 4, **caractérisé en ce que** le dispositif de commande (10) est implémenté dans au moins une des pièces d'appareil suivantes : appareil à main (20 ; 40) et un appareil de commande (9) couplé à l'appareil à main (20 ; 40).

6. Arrangement selon au moins l'une quelconque des revendications précédentes, **caractérisé en ce que** le dispositif d'exploitation (24 ; 44) ou, au choix, le dispositif de commande (10) est configuré de manière à empêcher le fonctionnement de l'appareil à main (20 ; 40) lorsqu'on constate lors de l'exploitation des moyens d'identification (31 ; 51) que le module à aiguille (22 ; 42) n'est pas agréé pour utilisation avec le module d'entraînement (21 ; 41).

7. Arrangement selon la revendication 3 ou au moins l'une quelconque des revendications 4 à 6, dans la mesure où ces dernières s'appuient sur la revendication 3, **caractérisé en ce que** le dispositif d'exploitation (24 ; 44) ou, au choix, le dispositif de commande (10) est configuré de manière à commander le fonctionnement de l'appareil à main (20 ; 40) conformément à l'une ou plusieurs des informations exploitables par le dispositif d'exploitation (24 ; 44) et pouvant être affectées au module à aiguille (22 ; 42).

8. Arrangement selon au moins l'une quelconque des revendications précédentes, **caractérisé en ce que** l'appareil à main (20 ; 40) est réalisé comme l'un des appareils à mains (20 ; 40) suivants : appareil à main de fond de teint permanent et de tatouage, appareil à main de décharge de matière active et appareil à main de vaccination cutanée.

9. Arrangement selon au moins l'une quelconque des revendications précédentes, **caractérisé en ce que** le module à aiguille (22 ; 42) est un module à aiguille à usage unique (22 ; 42).

10. Module à aiguille pour un arrangement selon au moins l'une quelconque des revendications précédentes, avec :
- un boîtier de module à aiguillle,
- des moyens de couplage sur le boîtier de module à aiguille qui sont configurés de manière coupler de manière amovible le boîtier de module à aiguille à un module d'entraînement (21 ; 41) avec un dispositif d'entraînement (4),
- un dispositif de perçage qui est disposé de manière décalable dans le sens longitudinal dans le boîtier du module à aiguille, et
- des moyens d'identification (31 ; 51) qui peuvent être exploités optiquement avec un dispositif d'exploitation (24 ; 44) affecté à un des moyens d'identification (31 ; 51),
étant donné que les moyens d'identification (31 ; 51) comprennent des moyens de masquage (32 ; 52) qui sont configurés de manière à masquer les signaux émis par un équipement émetteur (25 ; 45) réalisé comme source de lumière (27 ; 47), et
étant donné que les moyens de masquage (32 ; 52) comprennent un masque (33 ; 53) qui est configuré au moyen d'un cylindre (34 ; 54) avec des évidements (35 ; 55) de manière à masquer conformément au code affecté au module à aiguille (22 ; 42) les signaux émis par l'équipement émetteur (45 ; 65), étant donné que les évidements (35 ; 55) sont formés sur le cylindre (34 ; 54) pour laisser passer les signaux lumineux émis par la source de lumière (27 ; 47) à plusieurs capteurs de lumière (29) se trouvant sur la surface intérieure d'un anneau de capteurs (30 ; 50).
